# EUROPÄISCHE PATENTSCHRIFT

(11) **EP 0 669 953 B1**
(45) Veröffentlichungstag und Bekanntmachung des Hinweises auf die Patenterteilung: **10.06.1998**
(21) Anmeldenummer: 94928381.6
(22) Anmeldetag: 20.09.1994
(51) Int. Cl.: C08H 5/02, C08F 289/00, C12P 7/22

(54) **VERFAHREN ZUR HERSTELLUNG VON LIGNINHALTIGEN POLYMEREN**
PROCESS FOR PRODUCING LIGNIN-CONTAINING POLYMERS
PROCEDE DE PRODUCTION DE POLYMERES CONTENANT DE LA LIGNINE

(30) Priorität: 20.09.1993 DE 4331878
(43) Veröffentlichungstag der Anmeldung: 06.09.1995
(73) Patentinhaber: Hüttermann, Aloys, Prof. Dr., D-37075 Göttingen (DE)
(72) Erfinder: HÜTTERMANN, Aloys, D-37075 Göttingen (DE); MILSTEIN, Oleg, D-37075 Göttingen (DE)
(74) Vertreter: Harders, Gerhard, Dr.
(86) Internationale Anmeldenummer: EP9403142
(87) Internationale Veröffentlichungsnummer: WO9508588

(56) Entgegenhaltungen:
- EP-A- 0 275 544
- EP-A- 0 442 508
- DE-A- 3 037 992
- JOURNAL OF POLYMER SCIENCE, POLYMER CHEMISTRY EDITION, Bd.31, Nr.7, Juni 1993, NEW YORK US Seiten 1839 - 1846 A. M. BLINKOVSKY ET AL. 'Peroxidase-catalyzed synthesis of lignin-phenol copolymers'

## Beschreibung

Die vorliegende Erfindung betrifft ein Verfahren zur Herstellung von Polymeren, die Lignin und organische Verbindungen enthalten.

Aus der US-A 4 687 828 sind Pfropfcopolymere von Lignin-(2-propenamid)-(Na-2,2-dimethyl-3-imino-4-oxohex-5-en-1-sulfonat) und ein Verfahren zu deren Herstellung bekannt. Andere Polymere lassen sich nach diesem Verfahren nicht herstellen.

Aus der EP-A 91102118.6 sind Pfropfcopolymere aus Lignin und Acrylaten bzw. Methacrylaten bekannt. Sie müssen in einer Sauerstoff-freien Atmosphäre hergestellt werden. Nach diesem Verfahren lassen sich ausschließlich Derivate mit Acrylaten bzw. Methacrylaten herstellen.

Journal of Polymer Science, Polymer Chemistry Edition, Band 31, No. 7, June 1993, New York US, S. 1839-1846 offenbart, daß Horseradish-Peroxidase die Copolymerisation von Phenolen mit Kraftlignin in wäßrig-organischen Lösungsmitteln katalysiert. Da Peroxidase den Abbau von Lignin katalysiert, konnte der Fachmann erwarten, daß Peroxidase auch die Reversreaktion der Polymerisation beeinflußt. Es ist aber ausgesprochen überraschend, daß Peroxidase auch die Polymerisation von nicht-phenolischen Stoffen mit Lignin katalysiert.

Aufgabe der vorliegenden Erfindung ist es, ein Verfahren zur Herstellung von Polymeren aus Lignin und nicht-phenolischen organischen verbindungen zu schaffen, die auf einfache Weise hergestellt werden können und keine Sauerstoff-freie Atmosphäre benötigen.

Gelöst wird diese Aufgabe durch ein gattungsgemäßes Verfahren mit den Merkmalen des Kennzeichens des Hauptanspruchs.

Nach dem erfindungsgemäßen Verfahren lassen sich alle organischen Verbindungen mit Lignin umsetzen, die mindestens drei Kohlenstoffatome und mindestens eine Sauerstoff- und/oder Stickstoff- und/oder mindestens eine Mehrfachbindungsfunktion enthalten. Eine Sauerstoff-freie Atmosphäre ist für das Verfahren nicht erforderlich.

Das als Ausgangsmaterial verwendete Lignin kann natürliches Pflanzenlignin sein, beispielsweise aus Holz. Insbesondere wird aber Lignin eingesetzt, wie es bei der Celluloseproduktion in großen Mengen anfällt, beispielsweise in Form von Sulfitlignin oder Alkalilignin (Kraftlignin). Somit eröffnet die vorliegende Erfindung eine Möglichkeit zur Verwertung dieser bisher nur schwer verwertbaren Abfallprodukte.

Es hat sich gezeigt, daß sich organische Verbindungen mit weniger als 3 Kohlenstoffatomen nur schwierig mit Lignin polymerisieren lassen. Besonders einfach ist die Polymerisation mit Acrylderivaten oder mit organischen Verbindungen mit 5 oder mehr Kohlenstoffatomen.

Zur Polymerisation eignen sich insbesondere die folgenden Stoffe mit mindestens drei Kohlenstoffatomen:

Stoffe mit Sauerstoff-Funktionen, wie beispielsweise Carbonylverbindungen, insbesondere Aldehyde und Ketone; Hydroxylverbindungen, insbesondere ein- oder mehrwertige Alkohole; Etherverbindungen, insbesondere Einfach- oder Mehrfachether, Epoxyverbindungen.

Stoffe mit Stickstoff-Funktionen , wie beispielsweise Amine, Imine, Amide, Amidine, Nitrile, Isonitrile, Azoverbindungen.

Stoffe mit mindestens einer Mehrfachbindungsfunktion, wie beispielsweise Verbindungen mit mindestens einer Doppel und/ oder Dreifachbindung, vorzugsweise mit C-C-Doppelbindungen.

Die Verbindungen können auch mehrere der oben genannten funktionellen Gruppen aufweisen, wie es beispielsweise bei Carbonsäuren, Carbonsäureanhydriden, Säureamiden, Amidinen, Carbamiden, Carbonsäureestern, Peroxiden der Fall ist. Auch können zusätzliche weitere funktionelle Gruppen in den Verbindungen enthalten sein, wie beispielsweise bei Säurehalogeniden, Thioalkoholen und dergleichen.

Alle genannten Verbindungen können in Form aliphatischer, aromatischer, cycloaliphatischer oder heterocyclischer Verbindungen vorliegen.

Als radikalisch oxidierende Enzyme können die folgenden verwendet werden:

Peroxidasen, wie Mangan-Peroxidase, Rettich-Peroxidase; Phenoloxidasen, wie Laccase, Thyrosinase. Bei der Durchführung der Polymersationsreaktion müssen die Oxidationsmittel zugegen sein, die für die entsprechenden Enzyme die Substrate bilden. Bei der Verwendung von Peroxidasen ist das Oxidationsmittel Wasserstoffperoxid, bei der Verwendung von Phenoloxidasen ist es Sauerstoff. Weiterhin sind anorganische Peroxide und organische Peroxide und Hydroperoxide als Substrate geeignet. Jeweils verschiedene Enzyme und Substrate können als Mischungen zur Polymerisation eingesetzte werden.

Die Reaktion kann in wässrigen und/oder organischen Lösungsmitteln oder in mit solchen Lösungsmitteln gebildeten Dispersionen durchgeführt werden. Bei der Anwendung organischer Lösungsmittel ist es bevorzugt, die Enzyme in Form von Enzym-Matrix-Komplexen einzusetzen, wie sie in der DE 38 27 001 C1 beschrieben sind.

Nach dem erfindungsgemäßen Verfahren lassen sich alle organischen Verbindungen mit Lignin polymerisieren, die wenigstens 3, bevorzugt mehr als 5 Kohlenstoffatome aufweisen. Es lassen sich auch höhermolekulare Stoffe polymerisieren, beispielsweise Zucker, Stärke, Cellulosen, Hemicellulosen oder Derivate der genannten Stoffe, sowie synthetische Polymerisate. Lediglich in Fällen sterischer Hinderung kann die Polymerisation beeinträchtigt werden.

Die in der Polymerisation eingesetzten Lignine werden in dem verwendeten Lösungsmittel gelöst oder aufgeschlämmt, der Copolymeranteil zugegeben und die Mischung mit dem Enzym versetzt und das Cosubstrat, z. B. Sauerstoff bzw. Wasserstoffperoxid zugegeben. Bei Verwendung von Phenoloxidasen qenüqt häufig schon der im Reaktionsraum befindliche Luftsauerstoff zur Einleitung der Polymerisation. Die Reaktion läßt sich bei Zimmertemperatur durchführen. Bei höherer Temperatur wird sie beschleunigt, sodaß diese bis in einen Temperaturbereich von etwa 70 bis 80°C, bevorzugt bis etwa 60°C, durchgeführt werden kann. Weiterhin kann die Polymerisationsreaktion unter adiabatischen oder isothermen Bedingungen und bei erhöhtem oder vermindertem Druck vorgenommen werden. Bei Temperaturen über 80°C wird das Enzym im allgemeinen desaktiviert. Die Polymerisation ist im allgemeinen nach einigen Stunden bzw. Tagen beendet.

Die Erfindung wird nachfolgend anhand der Ausführungsbeispiele näher erläutert. Alle in den Beispielen enthaltenen Angaben werden als wesentlich für die Erfindung angesehen.

### Beispiel 1

### Polymerisation von Organosolvlignin mit Glucose

1g Organosolvlignin mit einem Molgewicht von ca. 6000 wurden in 20ml Wasser aufgeschlämmt und lg C¹⁴-markierte Glucose zugegebenDann wurde Laccaselösung zugegeben, bis eine Konzentration von 1000 U/ml in der Lösung erreicht war. Die Lösung wurde 4 Std. bei Zimmertemperatur polymerisieren gelassen.

Das gebildete Polymer wurde durch Einstellen eines pH-Werts von 2 mit Salzsäure ausgefällt und der gebildete Niederschlag in Alkali gelöst. Die Fällung mit Säure wurde wiederholt, bis die wässrige Phase keine Radioaktivität mehr zeigte.

Im entstandenen Polymerisat war die Glucose an das Lignin covalent gebunden. Der Nachweis wurde durch Messung der Radioaktivittät im Niederschlag geführt. Dazu wurde eine Gelchromatographie des Niederschlags an Sephadex LH 20 (Ausschlußgrenze 20.000) durcgeführt und eine Messung der Radioaktivität der hochmolekularen Fraktionen vorgenommen. Das Molekulargewicht des Copolymers lag oberhalb 20.000.

### Beispiel 2

Polymerisation von Organosolvlignin mit Vanillinsäure Entsprechend Beispiel 1 wurden lg Organosovlignin mit lg C¹⁴-markierter Vanillinsäure in Dioxan als Lösungsmittel polymerisiert. Das Enzym wurde in Form eines Matrix-Komplexes an Dextrangel vom Typ Sepharose CL 6B eingesetzt.

Das Polymerisat wurde wie in Beispiel 1 charakterisiert.

### Beispiel 3

### Polymerisation von Oragnosolvlignin mit Sorbit

Analog Beispiel 1 wurde Organosolvlignin mit Sorbit polymerisiert. Das entstandene Polymerisat wurde wie in Beispiel 1 charaketrisiert.

### Beispiel 4

### Polymerisation von Organosolvlignin mit Acrylamid

0,4 g Organosolvlignin wurde mit 3,2 g Acrylamid in Dioxan-Wasser 7:3 in Gegenwart von 1000 U/ml Laccase 3 Std. polymerisiert. Dabei wurde am Beginn der Reaktion 20 min lang Sauerstoff eingeleitet. Die Laccase wurde als Enzym-Matrix-Komplex wie in Beispiel 2 eingesetzt.

Das gebildete Polymerisat wurde durch Dialyse abgetrennt. Die löslichen Anteile wurden mit Salzsäure qefällt. Es wurden zwei Fraktionen erhalten. Eine Fraktion hatte ein Molgewicht über 12.000. Sie bestand aus 402 mg Polymerista mit einem Acrylatanteil von 75,1 %.
Die zweite Fraktion bestand aus 535 mg Polymerisat mit einem Molgewicht über 20.000 und einem Acrylatanteil von 14,8 %.

## Patentansprüche

1. Verfahren zur Herstellung von Polymeren, die Lignin und organische Verbindungen enthalten,
**dadurch gekennzeichnet, daß**
das Lignin mit organischen Verbindungen, die mindestens 3 Kohlenstoffatome sowie mindestens eine Sauerstoff- und/ oder Stickstoff- und/oder mindestens eine Mehrfachbindungsfunktion enthalten, mit Ausnahme von aromatischen Hydroxylverbindungen, in Gegenwart von radikalisch oxidierenden Enzymen und von Oxidationsmitteln, die für die entsprechenden Enzyme die Substrate bilden, polymerisiert wird.

2. Verfahren nach Anspruch 1, dadurch gekennzeichnet, daß als radikalisch oxidierende Enzyme Peroxidasen und/oder Phenoloxidasen eingesetzt werden.

3. Verfahren nach Anspruch 1, dadurch gekennzeichnet, daß als Oxidationsmittel Sauerstoff und/oder Wasserstoffperoxid eingesetzt werden.

4. Verfahren nach Anspruch 1, dadurch gekennzeichnet, daß als Verbindungen mit Sauerstoff-Funktionen Verbindungen mit Hydroxyl-, carbonyl- und/oder Etherfunktionen eingesetzt werden.

5. Verfahren nach Anspruch 1, dadurch gekennzeichnet, daß als Verbindungen mit Stickstoff-Funktionen Verbindungen mit Amin-, Imin-, Amid-, Nitril-. Isonitril- und/oder Azofunktionen eingesetzt werden.

6. Verfahren nach Anspruch 1, dadurch gekennzeichnet, daß als Verbindungen mit Mehrfachbindungsfunktion Verbindungen mit mindestens einer Doppel- und/oder Dreifachbindung, vorzugsweise mit C-C-Doppelbindung(en) eingesetzt werden.

7. Verfahren nach Anspruch 1, dadurch gekennzeichnet, daß die Polymerisation in einem wässrigen und/oder organischen Lösungsmittel durchgeführt wird.

8. Polymerisate aus Lignin und organischen Verbindungen, erhalten nach einem oder mehreren der Ansprüche 1 bis 7.

## Claims

1. Method of producing polymers containing lignin and organic compounds,
**characterised in that**
the lignin is polymerised with organic compounds containing at least 3 carbon atoms as well as at least one oxygen and/or nitrogen and/or at least one multiple bond function, with aromatic hydroxyl compounds being excepted, in the presence of enzymes for radical oxidation and of oxidants forming the substrates for the respective enzymes.

2. Method according to Claim 1, ***characterised in*** that peroxidases and/or phenol oxidases are used as enzymes for radical oxidation.

3. Method according to Claim 1, ***characterised in*** that oxygen and/or hydrogen peroxide are used as oxidants.

4. Method according to Claim 1, ***characterised in*** that compounds with hydroxyl, carbonyl and/or ether functions are used as compounds having oxygen functions.

5. Method according to Claim 1, ***characterised in*** that compounds with amine, imine, amide, nitrile, isonitrile and/or azo functions are used as compounds having nitrogen functions.

6. Method according to Claim 1, ***characterised in*** that compounds with at least one double and/or triple bond, preferably with C-C double bond(s), are used as compounds having a multiple bond function.

7. Method according to Claim 1, ***characterised in*** that the polymerisation is performed in an aqueous and/or organic solvent.

8. Polymerides of lignin and organic compounds, obtained according to any or several of the Claims 1 to 7.

## Revendications

1. Procédé à produire des polymères qui contiennent du lignine et des composés organiques,
**caractérisé en ce que**
le lignine est polymérisé par des composés organiques qui contiennent au moins 3 atomes de carbone ainsi qu'au moins une fonction d'oxygène et/ou d'azote et/ou au moins une fonction de liaison multiple, à l'exception des composés hydroxyles aromatiques, dans la présence des enzymes à oxydation radicalique et des oxydants qui constituent les substrats pour les enzymes respectifs.

2. Procédé selon la revendication 1, ***caractérisé en ce que*** des peroxydases et/ou des oxydases phénoliques sont utilisés en tant que des enzymes à oxydation radicalique.

3. Procédé selon la revendication 1, ***caractérisé en ce que*** de l'oxygène et/ou du peroxyde d'hydrogène sont utilisés en tant que des oxydants.

4. Procédé selon la revendication 1, ***caractérisé en ce que*** des composés aux fonctions hydroxyliques, carbonylique et/ou éthérique sont utilisés en tant que des composés aux fonctions d'oxygène.

5. Procédé selon la revendication 1, ***caractérisé en ce que*** des composés aux fonctions d'amine, d'imine, d'amide, de nitrile, d'isonitrile et/ou azoïques sont utilisés en tant que des composés aux fonctions d'azote.

6. Procédé selon la revendication 1, ***caractérisé en ce que*** des composés à au moins une liaison double et/ou triple, de préférence à (aux) liaison(s) C-C double, sont utilisés en tant que des composés à une fonction de liaison multiple.

7. Procédé selon la revendication 1, ***caractérisé en ce que*** la polymérisation est réalisée dans un solvant aqueux et/ou organique.

8. Polymères de lignine et des composés organiques, produits selon ne quelconque ou plusieurs des revendications 1 à 7.
